# EUROPEAN PATENT APPLICATION

(11) **EP 3 893 214 A1**
(43) Date of publication of application: **13.10.2021**
(21) Application number: 20168555.9
(22) Date of filing: 07.04.2020
(51) Int. Cl.: G08B 25/00

(54) **ALARM MANAGEMENT DEVICE**

(71) Applicant: Koninklijke Philips N.V., 5656 AG Eindhoven (NL)
(72) Inventor: LAUTE, Niels, 5656 AE Eindhoven (NL); GEURTS, Lucas Jacobus Franciscus, 5656 AE Eindhoven (NL)
(74) Representative: Philips Intellectual Property & Standards

(57) **Abstract**

The invention relates to an alarm management device (10) for managing an alarm (22) of a patient monitoring device (20). The alarm management device (10) comprises a sensor interface (12) configured to communicate with at least one sensor configured to detect a qualified user and determine a position of the qualified user in a room. The alarm management device (10) further comprises a device communication interface (14) configured to communicate with the patient monitoring device (20). The alarm management device further comprises at least one processor (16) configured to receive an alarm signal from the patient monitoring device (20), determine an alarm adaptation based on the position of the qualified user in the room, and send an alarm adapting signal to the patient monitoring device (20), wherein the alarm adapting signal indicates the determined alarm adaptation.

## Description

### FIELD OF THE INVENTION

The presently disclosed subject matter relates to an alarm management device, particularly for managing an alarm of a patient monitoring device, and a corresponding computer-implemented method. The presently disclosed subject matter further relates to a computer-readable medium.

### BACKGROUND OF THE INVENTION

The amount of alarms in intensive care unit (ICU) environments can become overwhelming for both patients and caregivers. On average, over 45 alarms occur per hour in an ICU. For patients, this can result in a delirium that carries a risk of developing into a chronic disease later in their recovery. Patients, family or other non-medical staff are not allowed to mute or dismiss an alarm - this must be done by medical staff (e.g. either in the room in which the alarm occurs or remotely). In order to avoid disturbing patients, this needs to be done quickly.

With the increasing amount of patients that need care and an ever-increasing amount of alarms, there is an increased potential for patients being disturbed (by their own alarms or those of other patients, for example) as well as a risk of caregivers losing track of these alarms. For example, the importance or intensity of an alarm may be missed due to the amount of alarms that may occur. This can develop into what is known as alarm fatigue, which puts patients at risk and can impact patient and/or staff satisfaction.

Current alarm implementations are tailored to do exactly this: with a sound and a graphical representation, a message is broadcast from the device itself to everyone in the room (or even everyone within earshot, for example). In some cases, alarms may be sent to a central control unit or to handheld devices used by staff. The alarms are designed to widely broadcast an alarm without having a specific target.

Even if a caregiver is in the room where an alarm is activated, they may not be able to mute or dismiss the alarm. In some cases, the caregiver may be farther away from the device, or occupied with a different task that requires their attention or their hands, for example cleaning the bed or helping the patient. In most cases, medical staff are asked to manually dismiss the alarm on the device, which can be cumbersome or slow, especially if the medical staff is not close to the device.

While an alarm is activated, for example with a sound or with a graphical alert, nearby patients, family and caregivers may be disturbed. There is therefore a clear benefit for an alarm management system that reduces the level of disturbance experienced by non-medical staff and which enables medical staff to acknowledge and mute (or, e.g., dismiss) an alarm more easily.

Published US patent application US 2018/023947 A1 describes a sound projection system which can guide medical staff to a source of an alarm intuitively. The sound projection system comprises a speaker configured to emit sound at a low frequency to improve the distance the sound wave can travel, without increasing the volume of the alarm. By using a plurality of strategically placed speakers with predetermined delays and adjustments based on their positions, a caregiver may be led to the source of the alarm, and in this way, the plurality of speakers effectively guides the caregiver. Additionally, the volume of an alarm may be adapted based on whether the device is in a quiet zone of the hospital, or based on time of day or ambient noise level, and a display of an alarm may be changed based on time of day or ambient light.

A problem of the prior art is that other patients and medical staff are still disturbed by the noise of alarms. The low-frequency speakers enables sound waves to travel through walls in order to be heard by a caregiver, but this also creates disruption for patients in neighboring or nearby rooms. Moreover, the placement of the speakers is crucial to the performance of the system, making installation and portability complex.

Having a multitude of loud and/or visibly distracting alarms causes disruption and distraction to patients, visitors (e.g. family members) and medical staff alike. For patients, being in an environment with disruptive noise or graphics can impede their recovery. For medical staff, the multitude of alarms can result in important alarms being overlooked in a sea of more minor alarms, and alarm fatigue may ensue. There is therefore a need to manage patient alarms to reduce disturbance to patients and medical staff, whilst ensuring that medical staff are aware of the alarms that are triggered.

### SUMMARY OF THE INVENTION

It would be advantageous to have an alarm management device which adapts an alarm based on a position of a qualified user in the room. An alarm management device and computer-implemented method are set out herein and are claimed that aim to address these and other concerns.

Existing alarm management systems and devices do not adapt to the presence or position of a qualified user in the room, and instead aim to improve audibility and/or visibility of an alarm based on ambient conditions or time of day. Even if a qualified user is in the same room as the alarm and has noticed the alarm, the alarm will continue to sound or display graphical warnings, and thus continues to disturb patients. It would therefore be advantageous to have an alarm management system configured to adapt an alarm of a patient monitoring device based on a position of a qualified user in the room. A technical problem addressed by the presently disclosed subject matter would be to improve alarm awareness in a medical environment whilst reducing patient disturbance.

The presently disclosed subject matter includes an alarm management device, a computer-implemented method and a computer-readable medium. The alarm management device may be configured to detect a qualified user and determine a position of the qualified user in a room using a sensor interface communicatively coupled to at least one sensor, and communicate with a patient monitoring device via a device communication interface. The alarm management device may be configured to receive an alarm signal from the patient monitoring device, determine an alarm adaptation based on the position of the qualified user in the room, and send an alarm adapting signal to the patient monitoring device, the alarm adapting signal indicating the determined alarm adaptation. For example, the alarm management device may detect a qualified user based on an RFID tag worn by the qualified user or by facial recognition, for example, and determine their position within the room. If the qualified user is far away from the device, the volume of the alarm may be increased, or directional speakers may be used to direct the alarm towards the qualified user's position, for example. If the qualified user is close to the device, the volume of the alarm may be reduced to avoid disturbing patients with an unnecessarily loud sound, for example. In these examples, a visual component of the alarm may similarly be adjusted based on the qualified user's position.

Embodiments help to improve a qualified user's alarm awareness, whilst reducing the disturbance caused to patients or others. Various examples and embodiments are provided herein describing how an alarm is adapted based on the position of the qualified user and how the qualified user can interact with the alarm from a distance.

In an embodiment, the alarm management device for managing an alarm of a patient monitoring device is provided. The alarm management device comprises a sensor interface configured to communicate with at least one sensor, a device communication interface and at least one processor. The at least one sensor is configured to detect a qualified user and determine a position of the qualified user in a room. The device communication interface is configured to communicate with the patient monitoring device. The at least one processor is configured to receive an alarm signal from the patient monitoring device, determine an alarm adaptation based on the position of the qualified user in the room, and send an alarm adapting signal to the patient monitoring device. The alarm adapting signal indicates the determined alarm adaptation. For example, the at least one sensor may determine that the qualified user is standing at a patient's bedside, in close proximity to the patient monitoring device. In this case, the alarm management device may determine to reduce a volume of an alarm from the patient monitoring device because the qualified user is so close, or the alarm management device may determine to reduce a text size of a warning message and provide detailed content that may otherwise not be present, again because of the proximity of the qualified user to a screen of the patient monitoring device.

By adapting the alarm to the position of the qualified user in a room, the alarm management device can prevent unnecessary disturbances of the patient that may result when an unnecessarily loud or bright alarm is used.

By detecting a qualified user, the alarm adaptation may be applied when a medical staff member is present and may not be applied otherwise. This may ensure that a qualified user (e.g. medical staff member, doctor, nurse etc.) is made aware of the alarm.

Aspects of the presently disclosed subject matter include a computer implemented method.

Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer.

In an embodiment, the computer program comprises computer program code adapted to perform all or part of the steps of an embodiment of the method when the computer program is run on a computer. Preferably, the computer program is embodied on a computer readable medium.

Another aspect of the presently disclosed subject matter provides a method of making the computer program available for downloading. This aspect is used when the computer program is uploaded into, e.g., Apple's App Store, Google's Play Store, or Microsoft's Windows Store, and when the computer program is available for downloading from such a store.

### BRIEF DESCRIPTION OF THE DRAWINGS

Further details, aspects, and embodiments will be described, by way of example, with reference to the drawings. Elements in the figures are illustrated for simplicity and clarity and have not necessarily been drawn to scale. In the Figures, elements which correspond to elements already described may have the same reference numerals. In the drawings,
Figure 1 schematically shows an example of an embodiment of an alarm management device,
Figure 2 schematically shows an example of an embodiment of an interaction between a qualified user and an alarm,
Figures 3A and 3B schematically show examples of embodiments of detecting the attention of the qualified user,
Figure 4 schematically shows an example of an embodiment of saccade detection,
Figure 5 schematically shows an example of an embodiment of alarm adaptation,
Figures 6A and 6B schematically show an example of an embodiment of alarm adaptation,
Figure 7 schematically shows an example of an embodiment of position determination,
Figure 8 schematically shows an example of an embodiment of a computer-implemented alarm management method,
Figure 9 schematically shows a computer readable medium having a writable part comprising a computer program according to an embodiment, and
Figure 10 schematically shows a representation of a processor system according to an embodiment.

### List of Reference Numerals:

- 10: an alarm management device
- 12: a sensor interface
- 12-1: an infrared sensor
- 12-2: a sensor
- 12-3: a sensor
- 12-4: a sensor
- 14: a device communication interface
- 16: at least one processor
- 20: a patient monitoring device
- 22: an alarm
- 30: a speaker
- 40: a display
- 50: a qualified user
- 52: a qualified user's eye
- 60: an infrared-reflective badge
- 62: an infrared-reflective region
- 64: a quick-response code
- 72: a fixation point
- 74: a saccade

### DETAILED DESCRIPTION OF EMBODIMENTS

While the presently disclosed subject matter is susceptible of embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described.

In the following, for the sake of understanding, elements of embodiments are described in operation. However, it will be apparent that the respective elements are arranged to perform the functions being described as performed by them.

Further, the presently disclosed subject matter is not limited to the embodiments, as features described herein or recited in mutually different dependent claims may be combined.

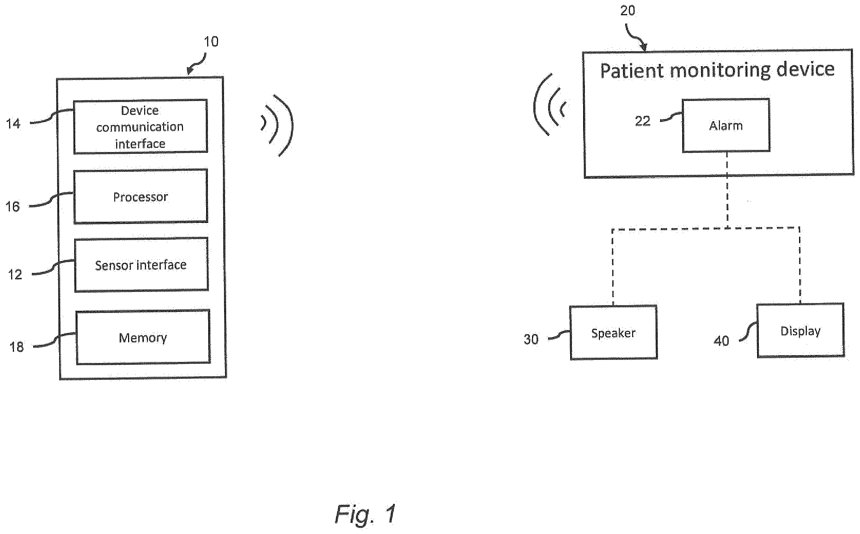

**Figure 1** schematically shows an example of an embodiment of an alarm management device 10. Alarm management device 10 comprise a sensor interface 12, a device communication interface 14 and at least one processor 16.

In an embodiment, the sensor interface 12 is configured to communicate with at least one sensor, the at least one sensor being configured to detect a qualified user. The at least one sensor may be configured to detect a qualified user based on the presence of an electronic tag, such as a radio frequency identification (RFID) tag, a swipe card indicating a level of authorization or qualification, a quick response (QR) code, a barcode, or the like. For example, the at least one sensor may comprise an RFID scanner and may be configured to detect the presence of an RFID badge worn by a nurse or doctor. Based on the presence of the RFID badge, the at least one sensor may determine that the wearer of the badge is a qualified user. For example, the RFID badge may indicate an authorization level, or the presence of the RFID badge alone may be an indication of authorization.

In an embodiment, the at least one sensor may be configured to detect a qualified user by first identifying a user and then determining if the identified user is a qualified user. The at least one sensor may therefore be configured to identify a user by means of, for example, facial recognition, biometric scanning, voice recognition or the like. If the alarm management device 10, using the at least one sensor and, for example, at least one processor 16 and/or access to an external or local memory such as memory 18, cannot identify the user, the alarm management device 10 may determine that the user is not a qualified user. If the alarm management device 10 can identify the user, the determined identity may be compared to a database, such as a whitelist or access list, indicating users who are qualified users. If the user is listed in the whitelist or access list, then the user is determined to be a qualified user.

In an embodiment, the at least one sensor is configured to determine a position of the qualified user in a room. The position of the qualified user in the room may be a relative position, for example relative to the sensor or relative to the alarm, or may be an absolute position, for example using a 2-D or 3-D coordinate system.

In an embodiment, the at least one sensor may be disposed on a wearable device, such as an augmented reality (AR) or mixed reality (MR) headset. The at least one sensor may be configured to calculate the user's location in the physical space of the room. Additionally or alternatively, the at least one sensor may be configured to identify the user, such as via an iris scanning mechanism, voice recognition, or by input from the user prior to or whilst wearing the wearable device (e.g. loading a profile).

In an embodiment, the at least one sensor may be configured to detect a user's attention on a particular object, such as a display. The at least one sensor may comprise an infrared (IR) scanner configured to detect at least one of a user's eye gaze and an IR-reflective badge. Throughout this disclosure, the term "infrared" is intended to refer to infrared (e.g. having a wavelength between 700 nm and 1 mm) as well as near-infrared light (e.g. having a wavelength between 780 nm and 2500 nm), and may also be referred to as active light. For example, the at least one sensor may comprise an eye tracking sensor configured to track a gaze of a user's eye by measuring an angle of reflection of infrared radiation from the user's cornea. In an embodiment, the at least one sensor may further track saccades of the user's eyes, which are rapid movements of the eye between fixation points. Saccade tracking may be used to detect, for example, if a user is reading or has read text, or to further enhance a gaze tracking process. In the example of the AR or MR headset, the gaze tracking component (e.g. the hardware and/or the software) may be embedded in the wearable device. For example, the user may be equipped with a wearable device configured to track their gaze and/or calculate the user's location in the physical space of the room. Moreover, the wearable device may be further configured to track the saccades of the user's eyes.

In an embodiment, the at least one sensor may comprise at least one of a camera, an IR sensor (e.g. IR scanner), an RFID scanner, a QR code reader, a barcode reader, a microphone, a biometric scanner and a facial recognition scanner. A camera may be configured to perform facial recognition. A microphone may be configured to perform voice recognition. The at least one sensor may comprise a sensor arranged to operate as a plurality of the above-listed sensors, such as an IR scanner with an integrated QR code reader, or a camera with an integrated microphone, etc. In an embodiment, the alarm management device 10 may be configured to communicate with at least one external sensor.

The at least one sensor may comprise a component of the alarm management device 10, and/or may comprise at least one external sensor. The at least one sensor may comprise a plurality of the above sensors, and may be disposed at various positions in the room. A plurality of sensors may communicate with each other and/or with the alarm management device 10 via the sensor communication interface 12, by means of a wireless and/or wired connection. For example, a plurality of sensors may be networked, such as in a wireless local area network (WLAN), a wired local area network (LAN), a Wi-Fi network or the like. As a further example, the plurality of sensors may be configured to communicate with each other and/or with the alarm management device 10 via wireless communication, such as Bluetooth, Wi-Fi, near field communication (NFC) or the like.

In an embodiment, the device communication interface 14 may enable the alarm management device 10 to communicate, either wiredly, wirelessly or using a combination thereof, with other devices. For example the device communication interface 14 may enable communication between the alarm management device 10 and the patient monitoring device 20, between the alarm management device 10 and one or more external sensors, between the alarm management device 10 and one or more external components (e.g. speakers), and/or between the alarm management device 10 and an external device, such as a wearable device of a qualified user.

The device communication interface 14 may be configured to communicate with any or all of the above external entities by using a computer network such as an internet, an intranet, a LAN, a WLAN, etc. The computer network may be the Internet. The computer network may be wholly or partly wired, and/or wholly or partly wireless. For example, the computer network may comprise Ethernet connections. For example, the computer network may comprise wireless connections, such as Wi-Fi, ZigBee, and the like.

In an embodiment, the alarm management device 10 may comprise at least one processor 16. The at least one processor 16 may be configured to receive an alarm signal from the patient monitoring device, for example via the device communication interface 14, and to determine an alarm adaptation based on the position of the qualified user in the room. For example, the at least one processor 16 may determine to direct an audible component of an alarm in the direction of the qualified user as determined by the at least one sensor, and/or may adjust a graphical representation (e.g. a font size, image size, font color, image color, font, formatting, positioning, and the like) and/or audio volume of an alarm based on a distance between the qualified user and the alarm source. That is, if the qualified user is close to a display displaying alarm text or graphics, the text or graphic size may be decreased, and in some embodiments, the amount of information displayed on the display may be increased. If, however, the qualified user is far away from the display, the text or graphic size may be increased for better visibility. In such a case, the amount of information, e.g. the amount of detail, may be decreased.

As a further example, if the qualified user is determined to be close to the alarm output, such as a speaker, a volume of an audible component of the alarm may be decreased. This may prevent unnecessary noise levels and thereby prevent (or mitigate) patient disturbance. If the qualified user is determined to be far away from the alarm output, the volume of the audible component of the alarm may be increased. In an example, the increase in volume may be accompanied by a directional sound emittance.

The alarm of the patient monitoring device 20 may comprise at least one of a visual component and an audio component. An alarm adaptation of the audio component may comprise at least one of adjusting a volume based on a distance between the qualified user and the audio output of the alarm and directing the audio component of the alarm towards the position of the qualified user. An alarm adaptation of the visual component of the alarm may comprise adjusting a display size, such as a text size or a graphic size, based on the distance between the display and the qualified user, adjusting an amount of information (e.g. detail) displayed on the display based on the distance between the display and the qualified user, a brightness of the display and/or a contrast of the visual component, based on the distance and/or position of the qualified user with respect to the display.

In an embodiment, the alarm adaptation may comprise pushing the alarm to a device worn or held by the qualified user. For example, an alarm may be forwarded to a wearable device such as a smartwatch, or a portable device such as a mobile phone, of the qualified user. The pushed alarm notification may comprise an audible component such as a sound, a visual alert such as a light or a text / graphical based alarm, and/or a haptic component. For example, the external device of the qualified user may vibrate to alert the qualified user. For example, an audible component of the alarm may be pushed to a wearable or portable device of the user, such as a Bluetooth headset. Bluetooth headsets are commonly used by healthcare staff. As a further example, the wearable device may be an AR or MR headset. One or more of the visual component and the audible component of the alarm may then be pushed to the wearable device. For example, a visual warning banner may be displayed to the user in the headset, e.g. in a mixed-reality display or a virtual reality environment.

In an embodiment, the alarm adaptation may be based on an attention of the qualified user on the alarm. For example, the alarm adaptation may be based on whether the qualified user has seen or noticed the alarm. The attention of the qualified user may be determined by detecting an eye gaze on the display of the alarm for a predetermined threshold of time (e.g. 3 seconds, 5 seconds, 10 seconds, etc.). The attention of the qualified user may, additionally or alternatively, be determined by detecting a reflection from an infrared-reflecting badge worn on the front torso of the user. For example, if the alarm management device 10 detects a reflection from the IR badge lasting more than a threshold time, the alarm management device 10 may determine that the qualified user is facing the alarm display and has thus become aware of the alarm display. In some examples, the attention of the user may be determined using voice or sound recognition, for example by identifying keywords or key phrases indicating that an alarm has been noticed.

When the attention of the qualified user is detected, the alarm adaptation may comprise a muting of the alarm and/or a temporary dismissal of the alarm (e.g. snoozing the alarm). For example, once the attention of the qualified user has been detected, the audio component of the alarm may be silenced for a predetermined amount of time, after which the audio component may occur again unless the alarm has been manually dismissed in the meantime. In another example, once the attention of the qualified user has been detected, the audio component and/or the visual component of the alarm may be switched off (e.g. snoozed) for a predetermined amount of time. The temporary dismissal of the alarm (either the audio component or the visual component, or both) may reduce patient disturbance by reducing the amount of noise emitted due to the alarm and/or reducing the amount of light emitted due to the alarm.

Any or all of the above-listed alarm adaptations may be used individually or in combination. In an embodiment, the type of alarm adaptation may depend on the type of alarm signal and/or the type of patient monitoring device.

In an embodiment, the alarm management device 10 may comprise a memory 18. The memory 18 may be implemented as an electronic memory, for example a flash memory, or magnetic memory, say hard disk or the like, or optical memory, e.g., a DVD. The memory 18 may comprise multiple discrete memories together making up the memory 18. The memory 18 may comprise a temporary memory, say a RAM. In the case of a temporary memory 18, memory 18 may be associated with a retrieving device to obtain data before use and to store the data in the storage, say by obtaining them over an optional network connection (not shown).

In an embodiment, the memory 18 may comprise a local memory and/or an external (e.g. remote) memory. For example, a list of qualified users may be stored internally in the device, or may be stored in an external memory and is merely accessed by the alarm management device 10.

The various components of the alarm management device 10 may be disposed within a single device, or may communicate with each other over a computer network. The computer network may be an internet, an intranet, a LAN, a WLAN, etc. The computer network may be the Internet. The computer network may be wholly or partly wired, and/or wholly or partly wireless. For example, the computer network may comprise Ethernet connections. For example, the computer network may comprise wireless connections, such as Wi-Fi, ZigBee, and the like. The subsystems may comprise a connection interface which is arranged to communicate with other subsystems of system 100 as needed. For example, the connection interface may comprise a connector, e.g., a wired connector, e.g., an Ethernet connector, an optical connector, etc., or a wireless connector, e.g., an antenna, e.g., a Wi-Fi, 4G or 5G antenna. The computer network may comprise additional elements, e.g., a router, a hub, etc.

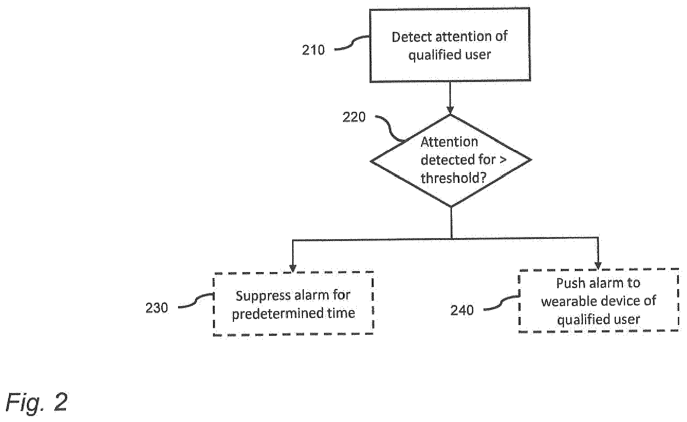

**Figure 2** schematically shows an example of an embodiment of an interaction between a qualified user and an alarm.

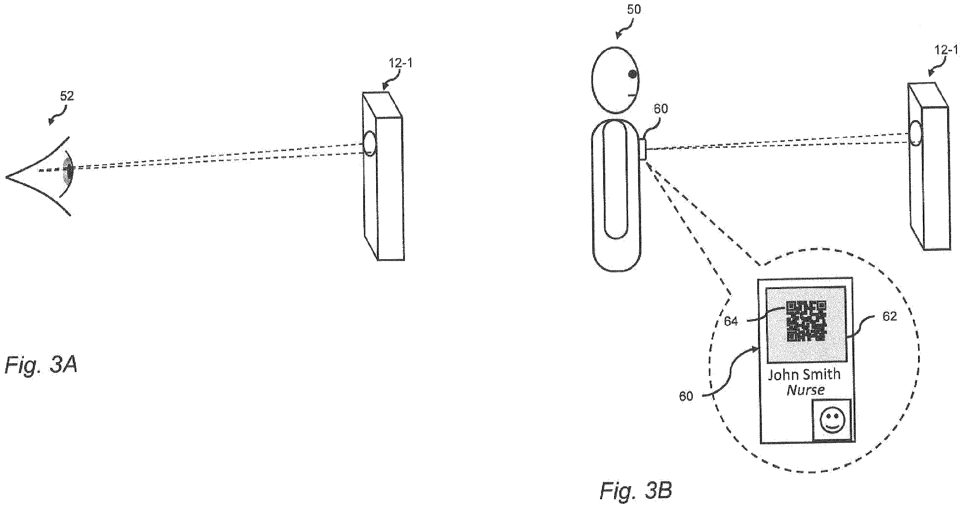

In an embodiment, the attention of the user may be detected in operation 210. The mechanisms for detecting the qualified user's attention will be elucidated with reference to Figure 3.

In an embodiment, the alarm management device 10 may be configured to determine whether the qualified user's attention is detected for at least a threshold amount of time, for example 3 or 5 seconds, in operation 220. In some examples, the use of a threshold amount of time may be replaced or adjusted by detecting keywords relating to the alarm in the speech of the qualified user.

In an embodiment, if the alarm management device 10 determines that the qualified user's attention has been detected for at least the threshold amount of time, the alarm management device 10 may determine to adapt the alarm by suppressing the alarm for a predetermined time, as shown in operation 230. Suppressing the alarm may comprise one or both of silencing an audio component of the alarm and disabling a visual component of the alarm.

In an embodiment, if the alarm management device 10 determines that the qualified user's attention has been detected for at least the threshold amount of time, the alarm management device 10 may, additionally or alternatively, determine to adapt the alarm by pushing the alarm to a wearable or portable device of the qualified user, as shown in operation 240. For example, the pushed alarm may display the same text and/or output the same noise as the alarm, or the pushed alarm may output different sound or different text. For example, a text of a pushed alarm may provide more or less detail than the text of the alarm. The pushed alarm may also comprise a haptic component, such as a vibration. In an example, the alarm may be both temporarily suppressed and pushed to the qualified user's device. In an embodiment, the pushed alarm may also be suppressed for the predetermined amount of time. For example, the qualified user may notice the alarm (e.g. the qualified user's attention is detected for the threshold time), and the alarm may be suppressed for 5 minutes and may be pushed to a wearable device worn by the qualified user. After 5 minutes, the wearable device worn by the qualified user may emit a visual alarm, an audio alarm and/or a haptic alarm. The alarm may, at this point, again be activated via the display 40 and/or the speaker 30.

In an embodiment, the alarm may be pushed to a device of the qualified user even if the attention of the qualified user has not been detected, for example in order to attract their attention without causing further disturbance to others.

**Figures 3A and 3B** schematically show examples of embodiments of detecting the attention of the qualified user. Figure 3A shows a first example in which a gaze of a qualified user's eye 52 is detected, and Figure 3B shows a second example in which an infrared-reflective badge worn on the front torso of the qualified user 50 is detected.

Referring first to Figure 3A, in an embodiment, the at least one sensor may comprise an infrared sensor 12-1. For example, the infrared sensor 12-1 may be configured to detect corneal reflections of the infrared light and center of the pupil, and by doing so tracks the eye gaze. In this eye-gazing technique, reflections of infrared light from the cornea are used, but there are multiple alternative techniques used in the art for eye gaze tracking, and any such technique may be used herein. In an embodiment, an individual may be determined to be a qualified user by using facial recognition, biometric sensing, self-identification or speech recognition, for example. The alarm management device 10 may therefore detect the attention of the qualified user if the qualified user's eye gaze is detected for at least a threshold gaze time.

Turning now to Figure 3B, in an embodiment, the infrared sensor 12-1 may be arranged to detect reflections of infrared light from an IR-reflective badge 60 worn by the qualified user 50. The infrared sensor 12-1 may be disposed in close proximity to the display 40 outputting the alarm. For example, the IR badge 60 may be provided to qualified users, to be worn on a front of their torso. Detecting reflections from the IR badge 60 may therefore indicate that the qualified user 50 wearing the IR badge 60 is facing the display 40, which may indicate that the qualified user 50 has become aware of the alarm. For example, the alarm management device 10 may detect the attention of the qualified user 50 if the IR badge 60 is detected (e.g. reflections from the IR badge 60 are detected) for at least a threshold gaze time. The threshold gaze time used for detecting the qualified user's attention via the IR badge 60 may be the same or different from the threshold gaze time used for detecting the qualified user's attention via corneal reflections as shown in Figure 3A. In an embodiment, both attention detection approaches may be used in conjunction in order to improve reliability, for example in case a qualified user's eyes are obscured and eye gaze detection is ineffective.

A face-on schematic of an IR badge 60 is also shown in Figure 3B. In an embodiment, the IR badge 60 may comprise an IR-reflective portion 62 and, optionally, a readable code such as a QR code 64. The QR code 64 may be disposed in the same region of the IR badge 60 as the IR-reflective portion 62, as shown, or the QR code 64 may be disposed elsewhere on the IR badge 60. For example, the infrared sensor 12-1 may be provided in combination with a QR code scanner, such as within a single scanning device. In an example, the QR code 64 may be invisible (e.g. cannot be seen by the naked eye), and may only be seen using infrared or near-infrared light. For example, the QR code 64 may be detected with near-infrared light and may be disposed within the IR-reflective portion 62.

The QR code 64 (or any other readable code, such as a barcode) may indicate a user's identity or an authorization level or occupation type (e.g. nurse, doctor) or the like.

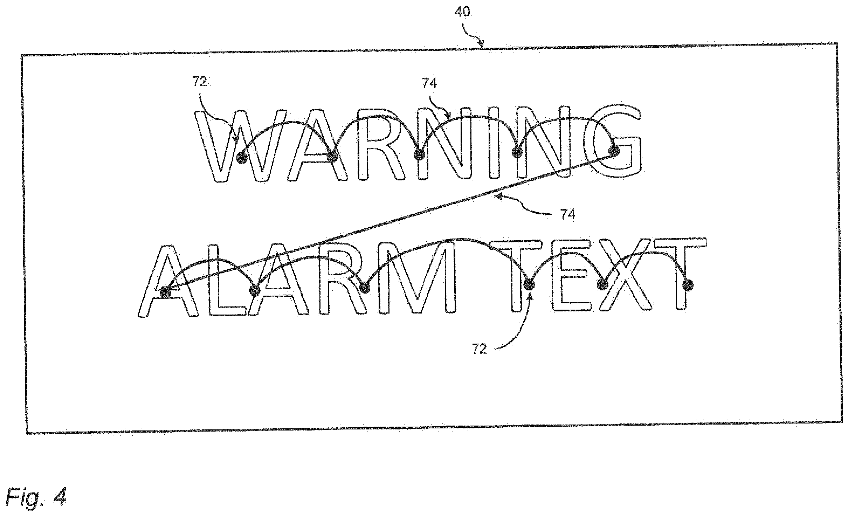

**Figure 4** schematically shows an example of an embodiment of saccade detection. In an embodiment, the at least one sensor may be arranged to detect saccades of a qualified user's eyes. A saccade refers to a quick eye movement between fixation points. Saccade tracking may be used to detect the motions of eyes during reading. In an embodiment, the alarm management device 10 may be configured to detect the qualified user's attention based on whether the qualified user has read the text of a visual component of the alarm. Determining whether the qualified user has read the text may be accomplished using, for example, saccade tracking. This process is illustrated in Figure 4.

Figure 4 shows an example of a visual component of an alarm, in this case reading "WARNING ALARM TEST", being displayed on display 40. Shown on the text of the visual component of the alarm are a plurality of fixation points 72 and saccades 74. Dots 72 represent fixation points - these are points at which the eye focuses, whereas lines 74 represent saccades, which represent the rapid eye movement between fixation points. The at least one sensor may comprise an infrared sensor, such as IR sensor 12-1, configured to track the saccades, e.g. the rapid eye movements, of a qualified user. When the at least one sensor determines that the qualified user has read the text of the alarm, for example by tracking the saccades and determining that the qualified user's eyes have followed the text displayed as part of the alarm by tracking the saccadic movements of the qualified user's eyes, the alarm management device may determine that the attention of the qualified user is detected.

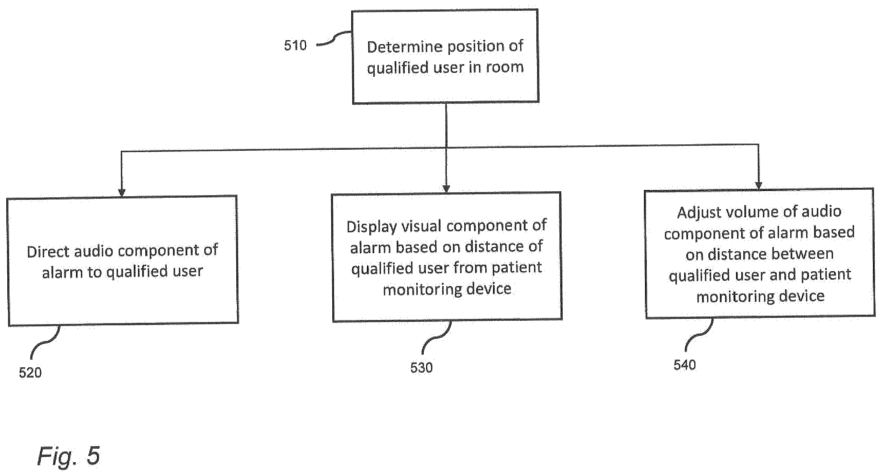

**Figure 5** schematically shows an example of an embodiment of alarm adaptation. These adaptations are merely exemplary and further adaptations, as discussed with reference to Figure 1, may additionally or alternatively be employed. These adaptations may be implemented in any combination or independently.

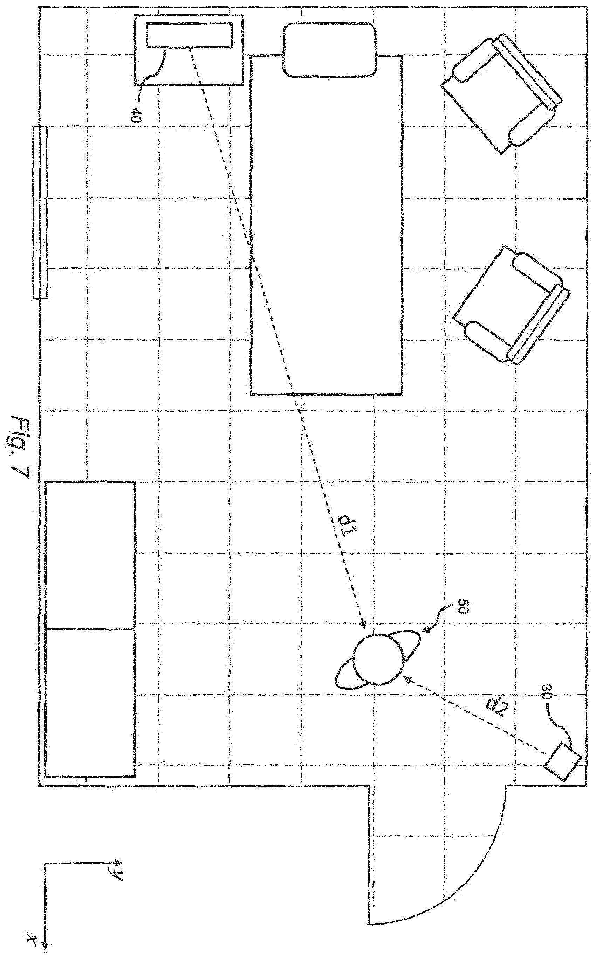

In operation 510, the alarm management device 10, via the sensor interface 12 communicating with the at least one sensor, determines the position of the qualified user 50 in the room. The position may be determined as an absolute position and/or a relative position, for example the position of the qualified user relative to the alarm management device and/or the alarm output device (e.g. speaker 30, display 40, etc.). The determination of position of the qualified user 50 will be elucidated with reference to Figure 7.

Based on the determined position of the qualified user, the alarm management device 10 may determine to adapt the alarm of the patient monitoring device by directing an audio component of the alarm to the position of the qualified user in operation 520. For example, the speaker 30 may comprise one or more directional speakers arranged to provide directed sound to a specified location. Directional speakers may target sound to a precise location, thereby preventing other patients, visitors or other staff from being disturbed. Any known type of directional speaker or directional speaker array may be used, such as a directional speaker that emits ultrasound waves using piezoelectric transducers.

Additionally or alternatively, the alarm management device 10 may determine to adapt the display of a visual component of the alarm based on the position of the qualified user, as shown in operation 530. For example, the display 40 may be rotated to face the qualified user. In another example, a size, such as a font size or a graphics size, may be increased if the qualified user is further away, and decreased if the qualified user is close to the display 40. The brightness, contrast and/or color of the visual component of the alarm may also be adapted based on the position (e.g. distance, absolute position or relative position) of the qualified user, and/or the ambient light conditions. For example, a brightness may be decreased if the ambient light is low (e.g. at night), and may be increased if the ambient light is high.

Additionally or alternatively, the alarm management device 10 may determine to adapt the audio output of the alarm based on a position of the qualified user, as shown in operation 540. For example, the volume of the audio component of the alarm may be increased if, for example, the qualified user is further away from the speaker(s), and may be decreased if the qualified user is close to a speaker. As a further example, if the alarm management device 10 comprises, or is communicatively coupled to a plurality of speakers configured to output the audio component of the alarm, and the plurality of speakers are disposed in different positions throughout the room, the alarm management device 10 may select to output the audio component from the speaker nearest to the position of the qualified user, or may adjust the volume of the audio component based on the distance between the qualified user and each speaker, such that the volumes of each speaker are independently determined.

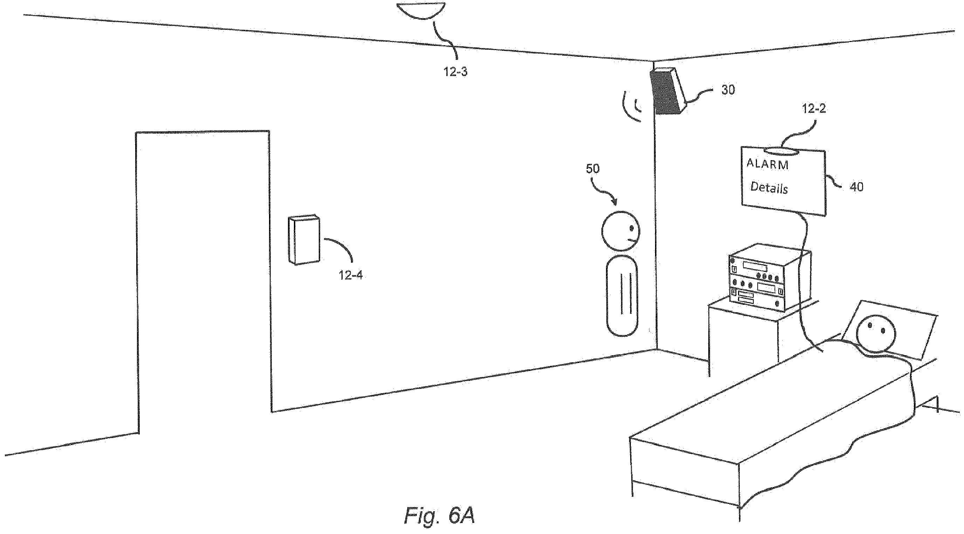

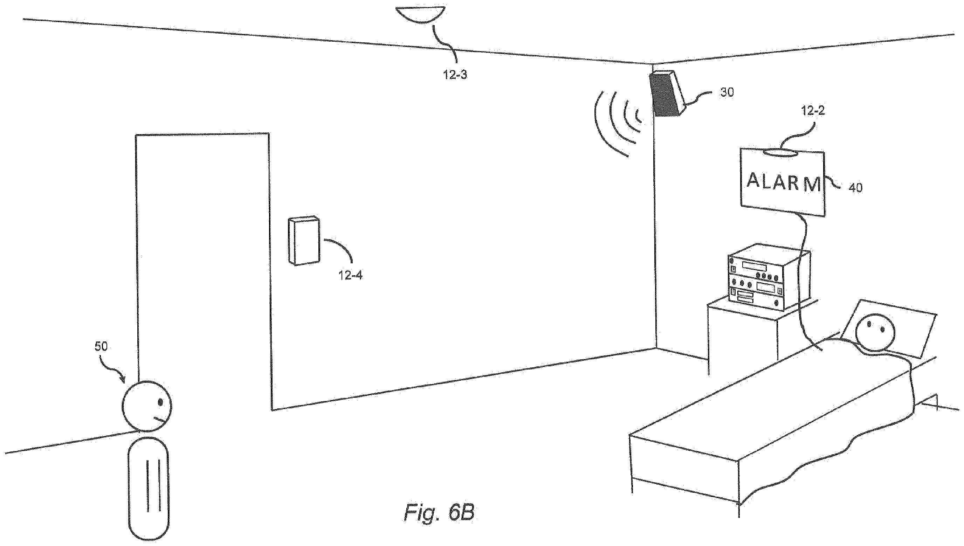

**Figures 6A** **and** **6B** schematically show an example of an embodiment of alarm adaptation. In Figure 6A, examples of alarm adaptations are shown based on determining that the qualified user is in close proximity to the display 40 and the speaker 30. In this example, the alarm management device 10 may comprise, or may be communicatively coupled to, at least one of sensors 12-2 and 12-3. For example, the at least one sensor may comprise one or both of sensors 12-2 and 12-3. Sensor 12-3 may be configured to determine an absolute position of the qualified user 50, for example using a predetermined coordinate or grid system, or a relative position of the qualified user 50, for example with respect to the display 40. Sensor 12-2 may be configured to detect an eye gaze of the qualified user 50, and/or perform facial recognition of the qualified user 50. For example, sensor 12-2 may be the same as sensor 12-1 described previously. Sensor 12-2 may be configured to determine the position of the qualified user 50 and/or the distance between the qualified user 50 and at least one of the display 40 and the speaker 30.

The alarm management device 10 may be communicatively coupled to an identification sensor 12-4 configured to detect a qualified user as they enter the room. In an embodiment, the identification sensor 12-4 may be a component of the alarm management device 10. The identification sensor 12-4 may be a biometric sensor allowing a qualified user to log their entry when they enter the room, or a near-field communication sensor, such as an RFID scanner, NFC scanner or the like, configured to detect the presence of a corresponding NFC or RFID tag worn or held by a qualified user 50. If the identification sensor 12-4 detects that a qualified user 50 has entered the room, the alarm management device 10 may be activated. In this way, the alarm management device 10 may not scan the room in search of a qualified user until the entrance of the qualified user into the room has been detected. This may reduce power consumption, improve privacy and security and/or improve system efficiency.

In Figure 6A, the qualified user 50 is determined to be close to both the display 40 and the speaker 30. As a result, the volume of the speaker 30 may be reduced and/or the text size shown on the display 40 may be reduced. Details pertaining to the alarm may additionally be shown on the display 40.

Conversely, Figure 6B shows the qualified user 50 further away from both the display 40 and the speaker 30. Consequently, the alarm management device 10 may determine to increase the volume of the speaker 30 and/or may increase the text size shown on the display 40, as illustrated.

**Figure 7** schematically shows an example of an embodiment of position determination. That is, Figure 7 is a top view of a room similar to that depicted in Figures 6A and 6B, for example from the perspective of sensor 12-3. Sensor-12-3 (shown in Figure 6) may be configured to determine distances between the qualified user 50 and one or more alarm output devices (e.g. speaker 30, display 40 etc.). For example, sensor 12-3 may determine a distance d1 between the display 40 and the qualified user 50, and a distance d2 between the speaker 30 and the qualified user 50. The distances d1 and d2 may be determined by first determining the absolute position of the qualified user 50, for example as (x, y) coordinates. In such a case, the absolute positions of the display 40 and/or the speaker 30 may be known to the alarm management device 10. Alternatively, the distances d1 and d2 may be determined by determining the qualified user's relative position, relative to each of the display 40 and the speaker 30. In an embodiment, a distance d1 between the qualified user 50 and the display 40 may be determined by a sensor, such as a proximity sensor, located on or near the display 40, and distance d2 between the qualified user 50 and the speaker 30 may be determined by a sensor, such as a proximity sensor, located on or near the speaker 30.

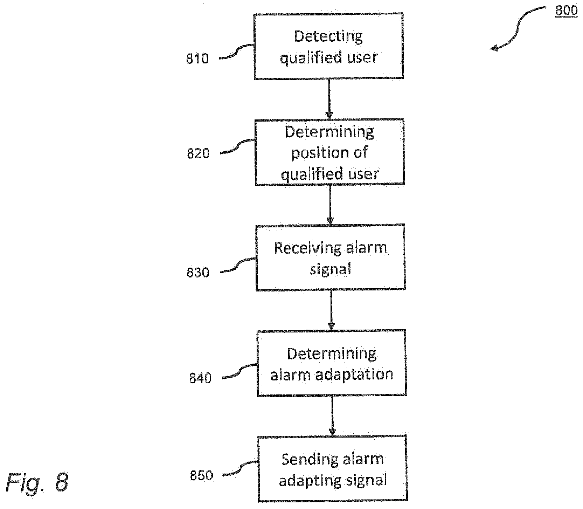

**Figure 8** schematically shows an example of an embodiment of a computer-implemented alarm management method 800. The method 800 may correspond to an operation of the alarm management device 10 of Figure 1. However, this is not a limitation, in that the method 800 may also be performed using another system, apparatus or device.

The method 800 may comprise, in an operation entitled "DETECTING QUALIFIED USER", detecting 810 a qualified user in a room. As discussed previously, detecting a qualified user may comprise identifying an individual and comparing their determined identity against a list of qualified users, and if the identified individual is listed in the list of qualified users, determining that the identified individual is a qualified user. Detecting a qualified user may additionally or alternatively comprise detecting an electronic tag, such as an RFID tag, QR code or the like, indicating that an individual is a qualified user, for example without the need for absolute identification. The at least one sensor previously described may be used to detect the qualified user.

The method 800 may further comprise, in an operation entitled "DETERMINING POSITION OF QUALIFIED USER", determining 820 the position of the qualified user in the room. The position may be determined as a distance from one or more device components to the qualified user, an absolute position (e.g. using coordinates), and/or a relative position (e.g. relative to one or more device components). For example, a qualified user may be determined to be distance d1 from a display 40 and distance d2 from speaker 30, as illustrated in Figure 7. As another example, the qualified user may be determined to be at a particular angle relative to one or more device component (e.g. display 40, speaker 30, etc.). As a further example, the qualified user may be determined to be at an absolute position such as a point in a coordinate system, for example as illustrated by the gridlines in Figure 7. The at least one sensor previously described may be used to determine the position of the qualified user.

The method 800 may further comprise, in an operation entitled "RECEIVING ALARM SIGNAL", receiving 830 an alarm signal from a patient monitoring device 20 comprising an alarm 22. The alarm signal may be received after the alarm has been triggered in the patient monitoring device 20. The alarm signal may indicate, for example, a type of alarm, a severity or priority of an alarm, or the like.

The method 800 may further comprise, in an operation entitled "DETERMINING ALARM ADAPTATION", determining 840 an alarm adaptation to apply to the alarm 22. The alarm adaptation may be based on the determined position of the qualified user. For example, the proximity of the qualified user to a display 40 and/or a speaker 30 may be used to adapt a visual component and/or an audio component of the alarm 22. Additional factors may also be used to determine the alarm adaptation, including but not limited to whether the qualified user's attention is detected, ambient light and noise conditions, alarm type, alarm priority or importance, and/or the availability of a portable or wearable device of the qualified user. For example, the alarm 22 may be pushed to a portable or wearable device worn or carried by the qualified user. Examples of alarm adaptations based on the position of the qualified user have been discussed throughout this disclosure, and any of which may be used in combination with any other alarm adaptation. For the sake of succinctness, these examples are not repeated here.

The method 800 may further comprise, in an operation entitled "SENDING ALARM ADAPTING SIGNAL", sending 850 an alarm adapting signal to the patient monitoring device 20. The alarm adapting signal indicates the determined alarm adaptation from operation 840. In an embodiment, the alarm 22 may be output via one or more of a display 40 and a speaker 30. In such an embodiment, the alarm adapting signal may be sent, for example with the alarm signal, to the alarm output device (e.g. speaker 30, display 40), without needing to be processed through the patient monitoring device 20. In other embodiments, the patient monitoring device 22 may be used to output the adapted alarm signal (e.g. the alarm signal adapted as indicated by the alarm adapting signal) to the alarm output device(s). The resulting alarm may then be adapted based on the position of the qualified user.

Many different ways of executing the methods shown in Figure 8 are possible, as will be apparent to a person skilled in the art. For example, the steps can be performed in the shown order, but the order of the steps may also be varied or some steps may be executed in parallel. Moreover, in between steps other method steps may be inserted. The inserted steps may represent refinements of the method such as described herein, or may be unrelated to the method. For example, a type of alarm adaptation may be at least partially determined before an alarm signal is received, such as based on the type of alarm. The operations of detecting a qualified user and determining their position may be performed simultaneously or in any order. Additionally, these operations may be performed after the alarm signal is received, for example. Moreover, a given step may not have finished completely before a next step is started.

Embodiments of the method may be executed using software, which comprises instructions for causing a processor system to perform the methods of Figures 5 through 8. Software may only include those steps taken by a particular sub-entity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units and/or parts of at least one of the systems and/or products set forth.

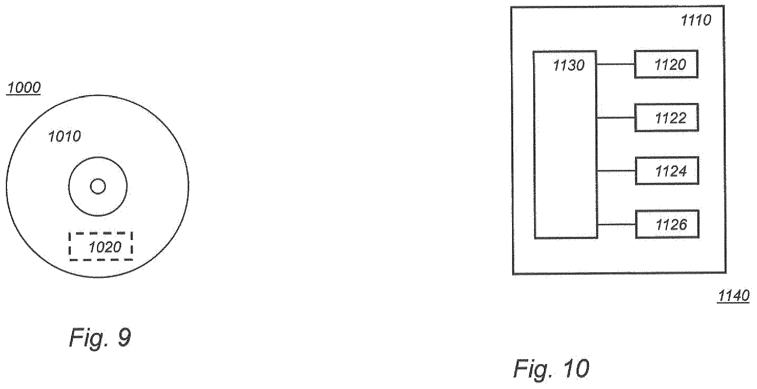

**Figure 9** shows a computer readable medium 1000 having a writable part 1010 comprising a computer program 1020, the computer program 1020 comprising instructions for causing a processor system to perform a method, such as the method of Figure 8. The computer program 1020 may be embodied on the computer readable medium 1000 as physical marks or by magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform said method of providing diagnosis support to a user.

**Figure 10** shows in a schematic representation of a processor system 1140, an example of the at least one processor 16, according to an embodiment of the alarm management device 10. The processor system comprises one or more integrated circuits 118. Circuit 118 comprises a processing unit 1120, e.g., a CPU, for running computer program components to execute a method according to an embodiment and/or implement its modules or units. Circuit 118 comprises a memory 1122 for storing programming code, data, etc. Part of memory 1122 may be read-only. Circuit 118 may comprise a communication element 1126, e.g., an antenna, connectors or both, and the like. Circuit 118 may comprise a dedicated integrated circuit (IC) 1124 for performing part or all of the processing defined in the method. Processor 1120, memory 1122, dedicated IC 1124 and communication element 1126 may be connected to each other via an interconnect 1130, for example a bus. The processor system 118 may be arranged for contact and/or contact-less communication, using an antenna and/or connectors, respectively.

For example, in an embodiment, processor system 1140, e.g., the alarm management device may comprise a processor circuit and a memory circuit, the processor being arranged to execute software stored in the memory circuit. For example, the processor circuit may be an Intel Core i7 processor, ARM Cortex-R8, etc. In an embodiment, the processor circuit may be ARM Cortex M0. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

While the alarm management device 10 is shown as including one of each described component, the various components may be duplicated in various embodiments. For example, the processor 1120 may include multiple microprocessors that are configured to independently execute the methods described herein or are configured to perform steps or subroutines of the methods described herein such that the multiple processors cooperate to achieve the functionality described herein. Further, where the device 10 is implemented in a cloud computing system, the various hardware components may belong to separate physical systems. For example, the processor 1120 may include a first processor in a first server and a second processor in a second server.

It should be noted that the above-mentioned embodiments illustrate rather than limit the presently disclosed subject matter, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of' when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claim.

## Claims

1. An alarm management device (10) for managing an alarm (22) of a patient monitoring device (20), the alarm management device (10) comprising:
- a sensor interface (12) configured to communicate with at least one sensor configured to:
- detect a qualified user;
- determine a position of the qualified user in a room;
- a device communication interface (14) configured to communicate with the patient monitoring device (20);
- at least one processor (16) configured to:
- receive an alarm signal from the patient monitoring device (20);
- determine an alarm adaptation based on the position of the qualified user in the room;
- send an alarm adapting signal to the patient monitoring device (20), the alarm adapting signal indicating the determined alarm adaptation.

2. The alarm management device (10) of claim 1, wherein the alarm comprises a visual component configured to be displayed on a display (40) communicatively coupled to the patient monitoring device (20), and wherein the at least one processor (16) is configured to detect an attention of the qualified user on the display (40).

3. The alarm management device (10) of claim 2, wherein if the attention of the user is detected, the alarm adaptation comprises suppressing an alarm for a predetermined time.

4. The alarm management device (10) of claim 2 or claim 3, wherein if the attention of the user is detected, the alarm adaptation comprises pushing an alarm message to an external user device.

5. The alarm management device (10) of any of claim 2 to claim 4, wherein the sensor interface (12) is communicatively coupled to an infrared (IR) sensor (12-1) configured to detect a gaze of the qualified user, and wherein the at least one processor (16) is configured to detect the attention of the qualified user on the display (40) by determining if the gaze of the qualified user is detected for at least a threshold gaze time.

6. The alarm management device (10) of any of claim 2 to claim 4, wherein the sensor interface (12) is communicatively coupled to an infrared (IR) sensor (12-1) configured to detect an IR-reflective badge worn by the qualified user, and wherein the at least one processor (16) is configured to detect the attention of the qualified user on the display (40) by determining if the IR-reflective badge worn by the qualified user is detected for at least a threshold gaze time.

7. The alarm management device (10) of claim 6, wherein the sensor interface (12) is communicatively coupled to a quick response (QR) code scanner configured to read a QR code identifying the qualified user, the QR code being disposed on the IR-reflective badge worn by the qualified user.

8. The alarm management device (10) of any one of claim 2 to claim 7, wherein the sensor interface (12) is communicatively coupled to a sensor configured to track saccades of the qualified user's eyes, and wherein the at least one processor (16) is configured to detect the attention of the qualified user on the display (40) based on the saccades of the qualified user's eyes.

9. The alarm management device (10) of any preceding claim, wherein the alarm (22) comprises an audible component configured to be output by at least one speaker (30) communicatively coupled to the patient monitoring device (20), and the alarm adaptation comprises at least one of: adjusting a volume of the audible component of the alarm (22) based on a distance between the qualified user and the at least one speaker (30), and directing the audible component of the alarm to the position of the qualified user.

10. The alarm management device (10) of any preceding claim, wherein the alarm (22) comprises a visual component configured to be displayed on a display (40) communicatively coupled to the patient monitoring device (20), and the alarm adaptation comprises at least one of: adjusting a size of the visual component of the alarm based on a distance between the qualified user and the display (40), adjusting an amount of information displayed on the display (40) based on the distance between the qualified user and the display (40) and adjusting a color of the visual component of the alarm based on an ambient light level in the room.

11. The alarm management device (10) of any preceding claim, wherein the sensor interface (12) is communicatively coupled to at least one of: a radio frequency identification (RFID) scanner, a quick response (QR) code reader, a camera configured to identify a face of the qualified user using facial recognition, and a biometric scanner.

12. The alarm management device (10) of any preceding claim, wherein the sensor interface (12) is communicatively coupled to a sensor configured to detect the qualified user upon entry to the room.

13. The alarm management device (10) of claim 12, wherein the at least one processor (16) is configured to receive the alarm signal if at least one sensor communicatively coupled to the sensor interface (12) detects that the qualified user has entered the room.

14. A computer-implemented method for managing an alarm (22) of a patient monitoring device (20), the method comprising:
- detecting a qualified user;
- determining a position of the qualified user in a room;
- receiving an alarm signal from the patient monitoring device (20);
- determining an alarm adaptation based on the position of the qualified user in the room;
- sending an alarm adapting signal to the patient monitoring device (20), the alarm adapting signal indicating the determined alarm adaptation.

15. A transitory or non-transitory computer-readable medium comprising data representing instructions which, when executed by a processor system, cause the processor system to carry out the method of claim 14.
